# EUROPEAN PATENT SPECIFICATION

(11) **EP 1 031 577 B1**
(45) Date of publication and mention of the grant of the patent: **16.05.2007**
(21) Application number: 00300532.9
(22) Date of filing: 25.01.2000
(51) Int. Cl.: C07K 14/415, C12N 15/82

(54) **Transformed Brassica plant**
Transformierte Brassicapflanze
Plante Brassica transformée

(30) Priority: 27.01.1999 GB 9901814
(43) Date of publication of application: 30.08.2000
(73) Proprietor: NORDDEUTSCHE PFLANZENZUCHT HANS-GEORG LEMBKE KG, 24363 Holtsee (DE)
(72) Inventor: Rahman, Muhammed Habibur, 4930 Maribo (DK); Jorsboe, Morten, 4800 Nykobing F (DK); Poulsen, Morten Helt, 4960 Holeby (DK)
(74) Representative: Harding, Charles Thomas

(56) References cited:
- WO-A-98/49889
- MENG JINLING ET AL: "The production of yellow-seeded Brassica napus (AACC) through crossing interspecific hybrids of B. campestris (AA) and B. carinata (BBCC) with B. napus." EUPHYTICA, vol. 103, no. 3, 1998, pages 329-333, XP009017372 ISSN: 0014-2336
- DATABASE WPI Section PQ, Week 199734 Derwent Publications Ltd., London, GB; Class P13, AN 1997-364291 XP002254510 & CN 1 108 874 A (SHAANXI FARMING SCI CENT), 27 September 1995 (1995-09-27)
- LIONNETON L. ET AL: 'Genetic analysis of agronomic and quality traits in mustard (brassica juncea)' THEOR APPL GENET vol. 109, 2004, pages 792 - 799
- RAHMAN M.H. ET AL: 'Development of yellow-seeded Brassica napus of double low quality' PLANT BREEDING vol. 120, 2001, pages 473 - 478
- CHOUDHARY B.R. ET AL: 'Interspecific hybridization between Brassica carinata and Brassica rapa' PLANT BREEDING vol. 119, 2000, pages 417 - 420
- DATABASE MEDLINE [Online] US NATIONAL LIBRARY OF MEDICINE (NLM), BETHESDA, MD, US 1989 AHUJA K.L. ET AL: 'Oil content and fatty acid composition of promising Indian Brassica campestris L. (Toria) genotypes' Database accession no. NLM2762244

## Description

The present invention relates to an organism. In particular, the present invention relates to a transformed plant, as well as tissues or cells thereof, and to transformed cells or tissues.

The *Brassica napus* (AACC, 2n=38) species is an amphidiploid species which originated in ancient times from the diploid species *B. campestris* (AA, 2n=20) and *B. oleracea* (CC, 2n=18) through interspecific hybridization. All of the naturally occurring strains of *B. napus* (which include oilseed rape) are of the black-seeded type.

In the *Brassica* species, the yellow seed is superior over the brown/black seed because of its higher oil and protein content, its lower fibre content and its aesthetic appearance in seed meal. For example, the yellow or partly yellow seed from *B. campestris* is associated with a 2-5% higher oil content, a 1% higher protein content and a 4-7% lower fibre content (Stringam *et al.* 1974; Daun and DeClercq 1988; Hu 1988; Simbaya *et al.* 1995); the yellow seed from *B. juncea* is associated with a 2.8% higher oil content, a 3.5% higher protein content and a 7.3% lower fibre content (Woods 1980; Simbaya *et al.* 1995); the yellow seed from *B. carinata* is associated with a 3.8% higher protein content and a 5.7% lower fibre content (Simbaya *et al.* 1995); and the seeds from the brown or yellow-brown seeded *B. napus* developed by cross breeding contain a 2-4% higher oil and protein content and a 3-7% lower fibre content (Shirzadegan and Röbbelen 1985; Hu 1988; Simbaya *et al.* 1995).

As most of the seed oil and protein are localised in the embryo, the seed coat contains very small amounts of oil and protein. Furthermore, in yellow seed, the proportion of seed coat to whole seed is 15% lower than that of the brown seed turnip rape *(B. campestris)* (Stringam *et al.* 1974). Thus, the higher oil and protein content in yellow seed is predominantly due to a larger proportion of embryo compared to the whole seed. The lower fibre content in meal derived from yellow or light coloured seed is largely due to a lower proportion of coat when compared to the whole seed (Stringam *et al.* 1974; Anjou *et al.* 1977).

The seed coat pigment of *Brassica* is composed mainly of polyphenols which are polymers of leucocyanidins (Leung *et al.* 1979; Hu 1988). The polyphenol is deposited in the palisade and crushed parenchyma layers of the seed coat (Vaugan 1970; Stringam *et al.* 1974). The palisade layer is the prominent cell layer of the seed coat and is assumed to yield a higher fibre content and a lower oil and protein content. This palisade cell layer is reduced from 1/2 to 2/3rd in the yellow seed of turnip rape (Stringam *et al.* 1974). Consequently, lower proportions of polyphenol and lignin are expected from the yellow seed (Anjou *et al.* 1977; Theander *et al.* 1977; Slominski *et al.* 1994). As a result, the digestibility of seed coat or meal from yellow seed is significantly higher than that from black seed (Bell and Shires 1982; Slominski *et al.* 1994).

In view of the aforementioned advantages associated with yellow seed, it would be desirable to produce transformed cells capable of yielding yellow colour seeds and/or yellow-seeded plants.

A transformed CC genome comprising an exogenous transparent seed coat gene obtained from an AA genome is taught herein. The transparent seed coat gene provides the yellow colour seed, as the coat is transparent thus enabling the yellow interior of the seed to be visualised. The visible yellow seed is produced by a substantially transparent seed coat which thereby exposes the natural embryo to the naked eye.

As used herein, the term "exogenous transparent seed coat gene" means a transparent seed coat gene having a different origin to other genes found on a particular genome.

The CC genome is found in some *Brassica*. Hence, the CC genome is obtainable from *Brassica*. Typically the CC genome is obtained from *Brassica*. Alternatively expressed the CC genome is a *Brassica* CC genome.

The AA genome is found in some *Brassica*. Hence, the AA genome is obtainable from *Brassica*. Typically the AA genome is obtained from *Brassica*. Alternatively expressed the AA genome is a *Brassica* AA genome.

Preferably the AA genome is obtained from any one of *Brassica campestris*, *Brassica napus* and *Brassica juncea*.

Preferably the AA genome is obtained from *Brassica campestris*.

Alternatively expressed, a transformed *Brassica* CC genome, wherein the transformed *Brassica* CC genome comprises a transparent seed coat gene obtained from a *Brassica* AA genome is taught herein.

Preferably the CC genome is a transformed *Brassica napus* genome.

The visible yellow seed is produced by a substantially transparent seed coat which thereby exposes the natural embryo to the naked eye. This is in direct contrast to the black seeded and yellow-brown seeded *Brassica* plant seeds which have darker colours due to dark colouration and dark pigmentation in the seed coat,

In accordance with the present invention, the term "transparent seed coat gene" - which may be interchanged with the term "transparent seed coat colour gene" or the term "yellow seed coat gene" or the term "transparent yellow seed coat gene" - includes one or more nucleotide sequences, but preferably at least two nucleotide sequences which are capable of imparting a transparent seed coat. If there are at least two nucleotide sequences, then they may be located on the same chromosomal locus or on different chromosomal loci.

Thus, the term "transparent seed coat gene" of the present invention can be alternatively stated as being a gene or a number of genes capable of making a seed coat substantially transparent and thereby enabling visualisation of the substantially natural yellow colour of the inner embryo component of the seed.

A seed coat is substantially transparent when there is a substantial absence of seed coat pigmentation when compared with the wild type seed coat.

As used herein, the term "wild type" means a form of a gene of allele that is considered the "standard" or most common type found in nature.

The present invention discloses a transformed *Brassica* plant, plant cell or plant tissue having a *Brassica* CC genome comprising an exogenous transparent seed coat gene obtained from an AA genome whereby the seeds obtained from said transformed plant, plant cell or plant tissue have a consistent and stable yellow colour.

Preferably the transformed plant, plant cell or plant tissue comprising an exogenous transparent seed coat gene is obtained from the AA genome from any one of *Brassica campestris*, *Brassica napus* and *Brassica juncea*.

Preferably the transformed plant, plant cell or plant tissue comprising an exogenous transparent seed coat gene is obtained from the AA genome of *Brassica campestris*.

Preferably, the transformed plant, plant cell or plant tissue is a transformed *Brassica napus* plant, plant cell or plant tissue.

In some embodiments, preferably the transformed *Brassica* plant is non-sterile (ie fertile).

Preferably the transformed *Brassica* plant, plant cell or plant tissue is capable of yielding seeds with a transparent seed coat or is capable of yielding plants having seeds with a transparent seed coat.

The present invention provides a method for increasing the levels of seed oil and protein and reducing the levels of fibre in a seed wherein the method comprising crossing a first *Brassica* plant, having a CC genome homozygous for a first transparent seed coat gene, said first transparent seed coat gene derived from a first AA genome of *Brassica campestris,* by application of an embryo rescue technique followed by *in vitro* culture, with a second *Brassica* plant having a second AA genome of *Brassica campestris* homozygous for a second transparent seed coat gene to produce an offspring *Brassica* plant heterozygous for said first and second transparent seed coat genes, and self-crossing the offspring *Brassica* plant to produce said yellow-seeded *Brassica* plant homozygous for said first and second transparent seed coat genes and wherein said yellow-seeded *Brassica* plant produces seeds having a consistent and stable yellow colour.

The method of the present invention may further comprise the optional steps of: (i) selecting yellow seeds from black seeds or brown seeds; and/or (ii) comparing levels of emcic acid and glucosinolate(s) between the yellow seeds and the black seeds and the brown seeds.

A transformed *Brassica napus* plant capable of yielding seeds with a transparent seed coat is also taught herein.

The present invention discloses a seed oil or a seed meal comprising an oil and protein content of at least about 70% seed dry matter and a fibre content of not more than about 8 % oil free meal.

Preferably the seed oil or a seed meal comprises an oil and protein content of from about 70% to about 80% seed dry matter.

Preferably the seed oil or a seed meal comprises a fibre content of not more than about 6% to about 8% oil free meal.

The present invention discloses a use of an AA genome as a vector for delivery of one or more genes of interest to an heterologous genome.

A transparent seed coat encoded by a transparent seed coat gene obtainable from NCIMB 40991 and/or NCIMB 40992 is taught herein.

Any one or more of the transformed plant, the transformed cell or the transformed tissue is prepared by use of the embryo rescue technique.

Preferably any one or more of the transformed plant, the transformed cell or the transformed tissue is prepared by a process which comprises a chromosome doubling step using an agent that causes chromosome doubling - such as colchicine.

Preferably any one or more of the transformed plant, the transformed cell or the transformed tissue is prepared by a process which comprises an embryo rescue step.

The present invention is advantageous in that it is now possible to produce yellow-seeded *Brassica* plants. The present invention is further advantageous in that it is now possible to produce yellow-seeded *Brassica napus* plants.

Thus, in accordance with the present invention, we have been able to prepare yellow-seeded transformed plants - such as transformed yellow-seeded *Brassica napus*.

In the present application the term "transformed" mean cells and plants that do not occur naturally but have, instead, been prepared by human intervention by selective cross-breeding, preferably a process that includes a biotechnological step (such as chromosome doubling and/or an embryo rescue step).

The above mentioned aspects of the present invention will now be described in more detail.

In the text, the term "yellow seed or yellow-seeded" means a visible yellow embryo produced by a substantially transparent seed coat.

In the text, the term "transparent" means a substantial absence of seed coat pigmentation when compared with the wild type seed coat.

In the text, the term "stable" means a yellow seed colour which is consistently trasmitted through subsequent generations.

Preferably, the seed colour is consistently trasmitted through at least two generations, preferably through at least three generations preferably through at least four generations, preferably through at least five generations, preferably through at least six generations, more preferably through at least seven generations.

In the text, the term "consistent" means a substantially even distribution of the yellow colour.

The term "low level" in relation to erucic fatty acid means a level less than 2% erucic acid.

The term "medium level" in reation to erucic fatty acid means a level greater than 2% erucic acid and less than 40% erucic acid.

The term "high level" in relation to erucic fatty acid means a level greater than 40% erucic acid.

The term "low level" in relation to glucosinolate(s) means a level of total glucosinolate(s) less than 25µmol/g seed.

The term "medium level" in reation to glucosinolate(s) means a level of total glucosinolate(s) greater than 25µmol and less than 40µmol per g seed.

The term "high level" in relation to glucosinolate(s) means a level of total glucosinolate(s) greater than 40µmol/g seed.

In arriving at the present invention, it was recognised that the main constraint of attempting to breed yellow-seeded *B. napus* plants that could produce yellow seeds was the lack of a transparent seed coat gene. More, in particular, it was recognised that there was an absence of a transparent seed coat gene in the CC genome of *B. napus*.

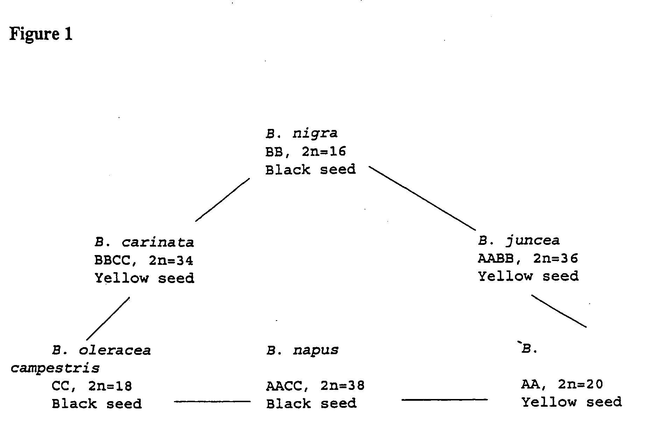

In part of the analysis of the present invention, which is represented by way of Figure 1, it was recognised that among the diploid *Brassica* species, yellow-seeded forms can only be found in the *B. campestris* (AA, 2n=20) species, whereas among the amphidiploid species, yellow-seeded variants can be found in *B. carinata* (BBCC, 2n=34) and in *B. juncea* (AABB, 2n=36). The yellow seed in these species is due to a transparent seed coat.

However, in accordance with the present invention it was surprisingly found that it was possible to transfer a transparent seed coat gene into a plant, such as into its genome, that lacked such a gene.

The transfer can occur via selective cross-breeding procedures which incorporate certain essential or preferred technical features. Details of these techniques and procedures are presented later on.

Up until the present invention, interspecific crosses in *Brassica* have been carried out in different directions to exploit the yellow-seeded natural variants of *B. campestris*, *B. juncea* and *B. carinata* with a view to breeding yellow-seeded *B. napus*. So far, only limited success has been achieved. By way of example, Barcikowska *et al.* (1987) and Zaman (1988) attempted to develop a yellow-seeded CC genomic species using the interspecific cross of {yellow-seeded *B. carinata* x black-seeded *B. alboglabra*/*B. oleracea*} as an intermediate step in breeding yellow-seeded *B. napus* but in these studies only partly yellow seed was obtained.

Liu (Liu 1983; Liu and Gao 1987) reported on an attempt to breed a yellow seeded *B. napus* developed from the {*B. napus* x *B. campestris* (*B. chinensis*)} cross. However, the frequent occurrence of black seed and/or black spots on the coat, as well as the change in seed coat colour due to environmental factors, indicated that their yellow seeded strains were not stable over generations.

Zaman (1988) isolated a brown-seeded strain from an interspecific cross of {(*B. carinata* x *B. campestris*) x *B. napus*}. Chen (Chen *et al* 1988; Chen and Heneen 1992) obtained yellow seed in an F₂ population derived from a cross between the resynthesised *B. napus* (resynthesised from *B. campestris* and a partly yellow-seeded *B. alboglabra*) and a yellow-brown-seeded *B. napus* breeding line. However, the yellow seed character did not breed true - such as up to F₄ generation. Chen's partly-yellow-seeded *B. alboglabra* was developed from an interspecific cross of yellow-seeded *B. carinata* and black-seeded *B. alboglabra*.

Rashid *et al.* (1994) reported on their attempt to develop yellow-seeded plants from a more complex interspecific cross of {[(*B. napus x B. juncea*) x *B. napus*] x [(*B. napus* x *B. carinata*) x *B. napus*]}. However, the stability of the seed coat colour in the resulting hybrids was not reported.

Van Deynze (Van Deynze *et al.* 1993; Van Deynze and Pauls 1994) reported on a partly yellow-seeded *B. napus* plant which were developed in the University of Manitoba/Guelph. However, an unstable yellow seed colour and a significantly high seed coat pigmentation (black/dark brown seed) was observed when the yellow-seeded strains were grown at a lower temperature of 16°C/12°C (day/night). This instability of yellow seed colour may render such varieties unsuitable for some major *B. napus* growing areas such as Northern Europe and North America.

Tang *et al.* (1997) developed yellow-seeded *B. napus* lines from different breeding approaches, such as from interspecies crosses of {(*B. campestris* x *B. oleracea*) x *B. napus};* {*B. napus* x *B. juncea}; {B.napus* x *B. campestris};* from irradiated progenies of *B. napus*; and from progenies of intervarietal crosses of *B. napus*. However, the stability of the yellow seed colour was not reported.

It is apparent that transparent seed coat gene of *B. campestris*, *B. juncea* and *B. carinata* have been utilised in the prior art in different ways with a view to breeding yellow-seeded *B. napus* plants.

In contrast, our invention relates *inter alia* to the transfer of the transparent seed coat gene *via* the AA genome of Yellow Sarson (*B. campestris*) into the CC genome of *B. napus*. Indirect evidence is provided that the transfer of the transparent seed coat gene is achieved through allosyndesis between the A- and the C-genome chromosomes. The resultant *B. napus* strain, carrying the transparent seed coat gene of Yellow Sarson (*B. campestris*) in its CC genome, was used in breeding yellow-seeded oilseed rape.

By way of further background information, Attia (Attia and Röbbelen 1986; Attia *et al.* 1987) studied meiotic configuration of chromosomes in: (i) amphihaploids AC, AB and BC which were generated from crosses between three elementary diploid species and (ii) digenomic triploids AAC, ACC, BBC and BCC which were generated from crosses between the amphidiploid and the diploid species. In the amphihaploids AC 12.3 chiasmata (7.3 II) was found. In AB 5.8 chiasmata (4.36 II) was found and in BC 2.0 chiasmata (1.9 II) was found. In the digenomic triploids BBC and BCC, there was a predominance of preferential pairing between the two homologous genomes while the third single genome remained unpaired. On the other hand, in the AAC and the ACC triploids, a high frequency of allosyndetic pairing between the A and the C genome was expressed by the formation of one or more trivalents in over 50% of the pollen mother cells. According to Busso *et al.* (1987), the homologous pairing between the A and the C genome chromosomes in trigenomic haploid (ABC) was not disturbed by the presence of the B genome chromosomes.

These cytological observations clearly suggested that the *Brassica* A and C genome chromosomes are more closely related to each other, while the B genome chromosomes are phylogenetically distant from the A and the C genome chromosomes. Therefore, in amphihaploid genome constitution, it was recognised the transfer of gene(s) from the A-to the C-genome or *vice versa* could occur relatively easily.

One technique for preparing the transformed plants - namely by use of the AA genome as a vector for the transparent seed coat gene - is presented in the following examples.

In a highly preferred embodiment, the present invention is based on our finding that it is possible to use the transparent seed coat gene of the present invention to prepare transformed cells.

In addition, the present invention also discloses transformed plants comprising the transparent seed coat gene of the present invention.

The following samples were deposited in accordance with the Budapest Treaty at the recognised depositary of The National Collections of Industrial and Marine Bacteria Limited (NCIMB) at 23 St. Machar Drive, Aberdeen, Scotland, United Kingdom, AB24 3RY on 7th December 1998.
*Brassica napus* 13-217 - deposit number NCIMB 40991
*Brassica napus* 13-219 - deposit number NCIMB 40992

The present invention will now be described only by way of example, in which reference is made to the following Figures:
Figure 1 which is a schematic diagram;
Figure 2 which is a schematic diagram;
Figure 3 which is a schematic diagram;
Figure 4A which is schematic diagram;
Figure 4B which is schematic diagram;
Figure 5A which is schematic diagram;
Figure 5B which is schematic diagram;
Figure 6A which is a photographic representation;
Figure 6B which is a photographic representation;
Figure 7 which is a schematic diagram; and
Figure 8 which is a photographic representation.

In more detail, Figure 1 is a schematic diagram showing the occurrence of yellow seed in the *Brassica* species.

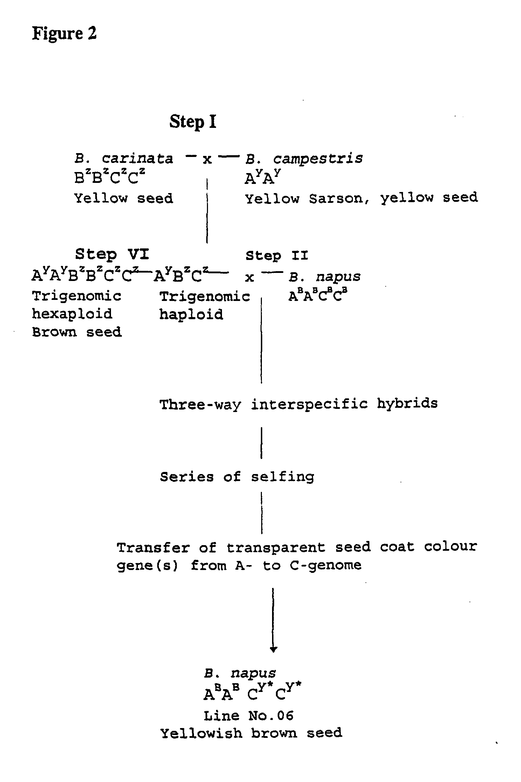

Figure 2 is a schematic diagram showing the approach taken for transferring the transparent seed coat gene from the A- to the C- genome. The normal size capital letters A, B and C indicate the three *Brassica* genomes. The superscripted letter y indicates the transparent seed coat gene of Yellow Sarson (*B. campestris*). The superscripted letter z indicates the transparent seed coat gene of *B. carinata* which are of different genomes relative to the transparent seed coat gene of Yellow Sarson (*B. campestris*). The superscripted letter B indicates the black seed coat gene of natural *B. napus*. The symbol * indicates the transfer, through allosyndesis, of the transparent seed coat genes from the A - to the C - genome.

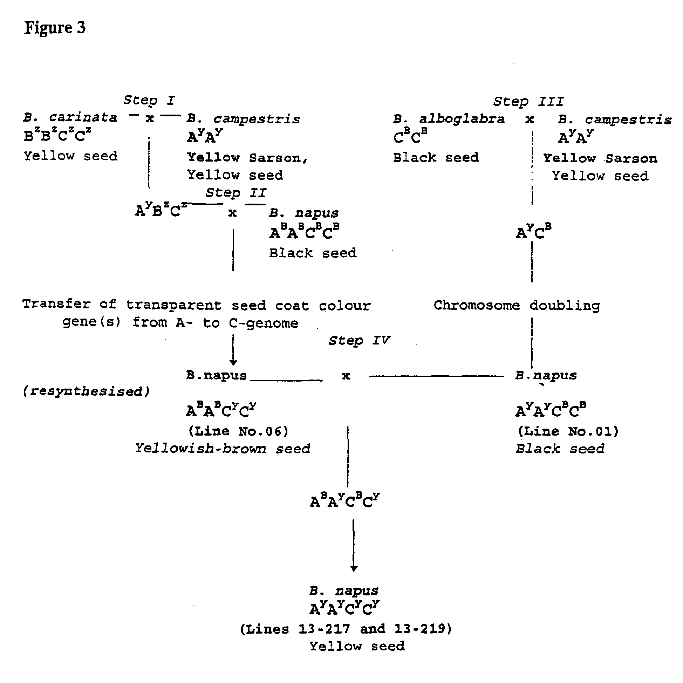

Figure 3 is a schematic diagram showing the process of development of the yellow seeded *B. napus* from the interspecific crosses. The genomic and seed coat gene designations are as described in Figure 2.

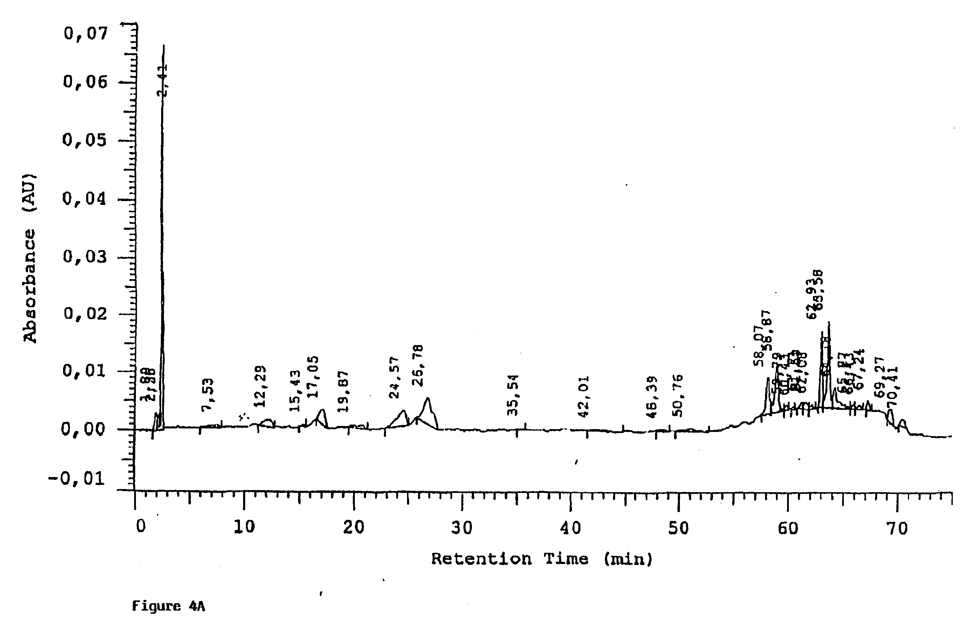

Figure 4A is a HPLC chromatogram of alkaline hydrolysate of transparent seed coat (11A5156.082-K1217) of *Brassica napus* measured at 280nm.

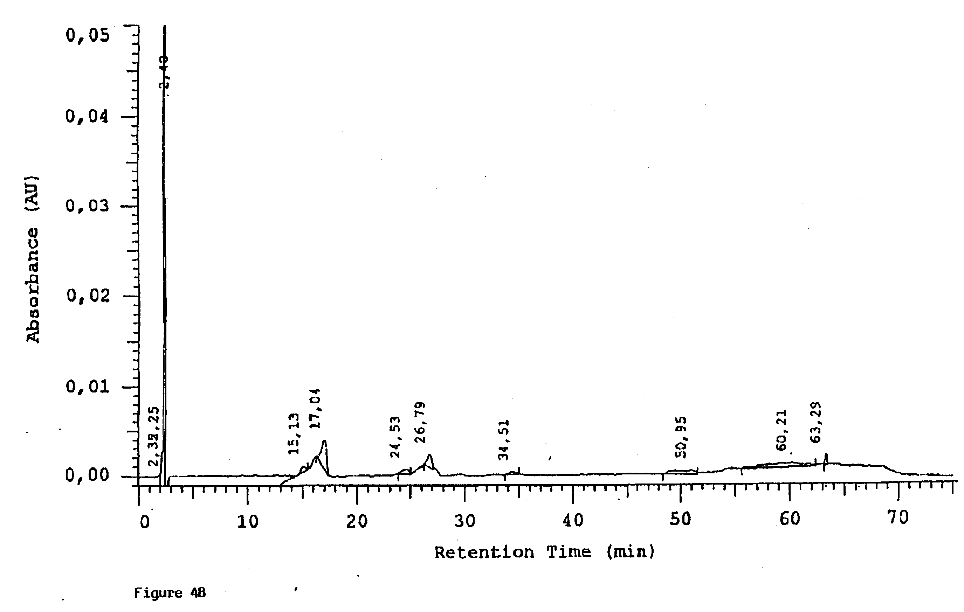

Figure 4B is a HPLC chromatogram of alkaline hydrolysate of transparent seed coat (11A5156.082-K1217) of *Brassica napus* measured at 360nm.

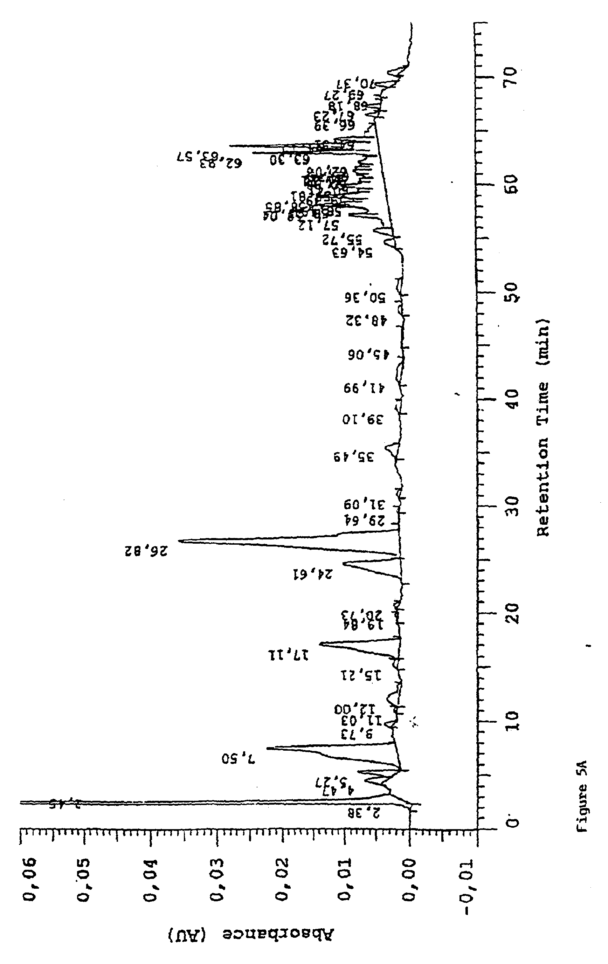

Figure 5A is a HPLC chromatogram of alkaline hydrolysate of black seed coat (Miro) of *Brassica napus* measured at 280nm.

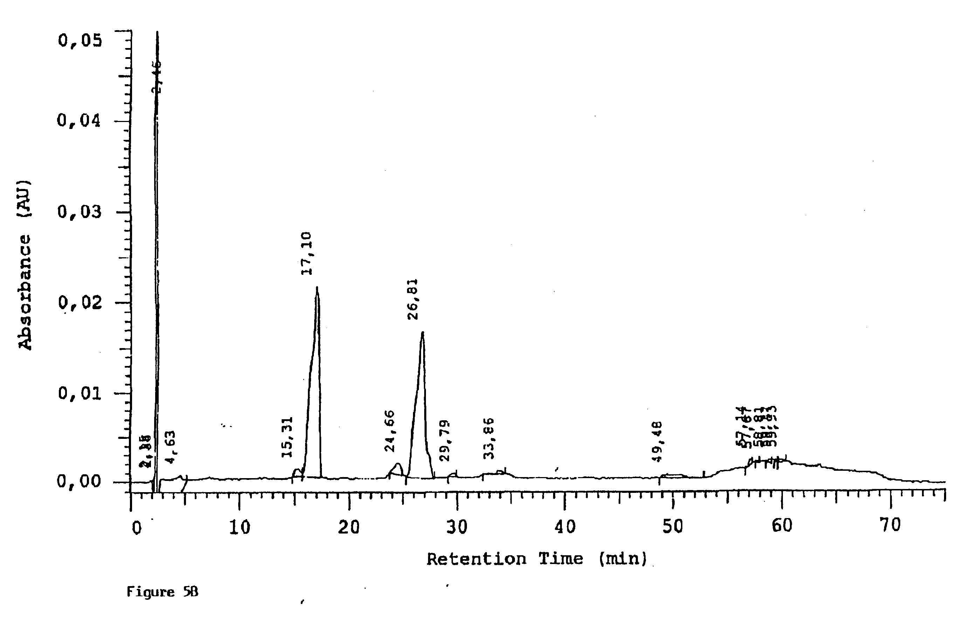

Figure 5B is a HPLC chromatogram of alkaline hydrolysate of black seed coat (Miro) of *Brassica napus* measured at 360nm.

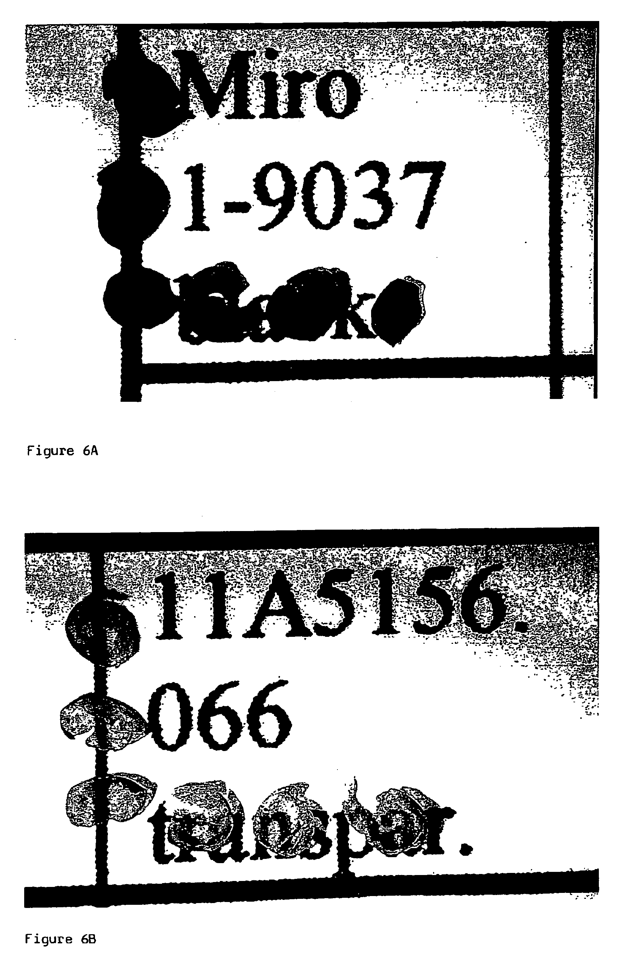

Figure 6A is a photographic representation of seed coats from black seeds of *B.napus*.

Figure 6B is a photographic representation of seed coats from yellow seeds of *B. napus*. The yellow seeds have a transparent seed coat.

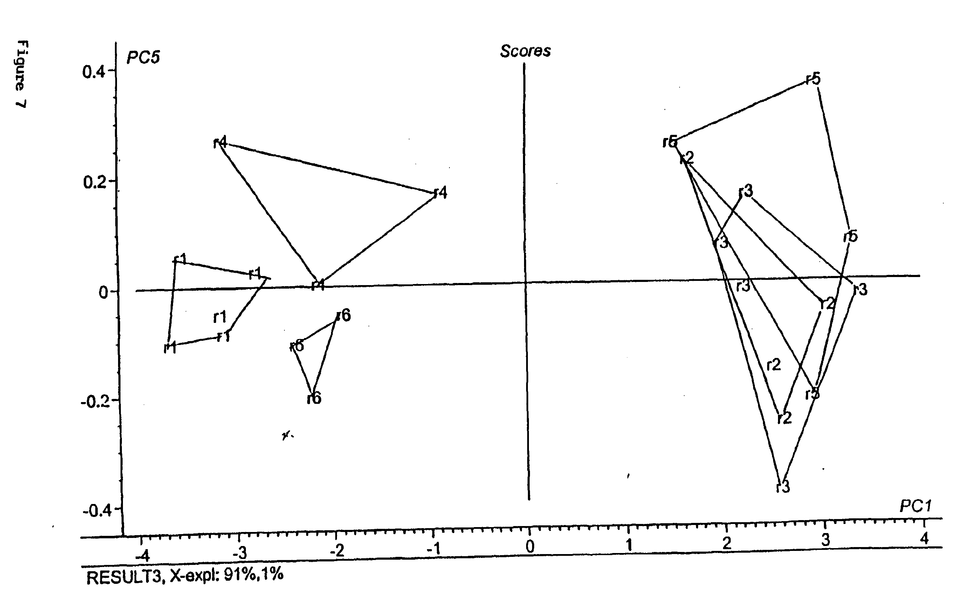

Figure 7 is a Principal Component Analysis (PCA) plot of Agrovision colour data from six seed samples (dark and yellow seeds) of *Brassica napus*.

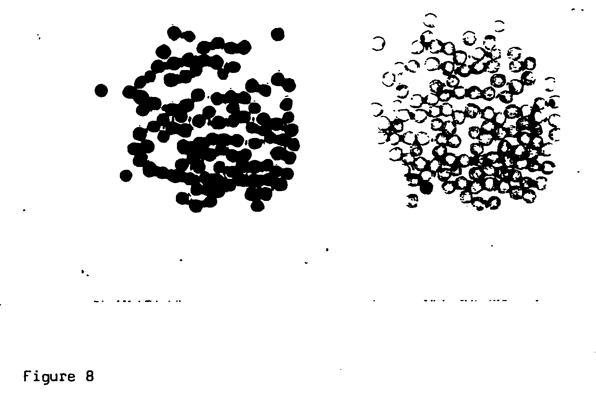

Figure 8 demonstrates the colour difference between yellow seed (from the present invention) and black seed (conventional type) of *Brassica napus*.

### METHODS AND PROCEDURES

The complete procedure of the present invention is given stepwise in Steps I-VI.

Steps I-II (which are summarized in Figure 2) involve the generation of yellowish brown seeded *B. napus* plants carrying the transparent seed coat genes of Yellow Sarson (*B. campestris*) in its CC genome.

Step III (which is summarised in Figure 3) involves the resynthesis of the *B. napus* line carrying the transparent seed coat genes of Yellow Sarson (*B. campestris*) into its AA genome.

Step IV (which is also summarized in Figure 3) involves the development of the yellow seeded *B. napus* using the materials developed in Step I-II and Step III.

Step V involves the development of yellow-seeded *B. napus* of double low quality by crossing yellow-seeded lines with conventional *B. napus* varieties of double low quality.

Step VI involves the generation of hexaploids which supports the claim of the present invention.

### Step I: Interspecific Cross

### Yellow-seeded B carinata x Yellow-seeded Yellow Sarson (B. campestris)

An interspecific cross between the yellow-seeded *B. carinata* (BBCC) line (Accession No. 381078) and the yellow-seeded Yellow Sarson (*B. campestris*) (AA) line of Bangladesh origin (Accession No. 3-0166.001) was carried out and trigenomic haploid plants (ABC) were generated (Figure 2).

### Step II: Interspecific Cross

### Trigenomic F₁ hybrid (ABC) x Black-seeded B. napus (AACC)

Trigenomic haploids (ABC) of Step I were crossed with black-seeded natural *B. napus* (Accession No. 1-9007) and the resultant three-way interspecific hybrids were selfed for a number of generations (Figure 2). These interspecific crosses were designed to ensure allosyndesis between the A- and the C-genome chromosomes in the trigenomic haploids (ABC) and subsequent selfed generations would transfer the transparent seed coat gene from the A- to the C-genome.

Steps I-II resulted in the yellowish-brown seeded *B. napus* line No. 06.

### Step III: Interspecific Cross to resynthesise B. napus

### (Black-seeded B. alboglabra x Yellow-seeded Yellow Sarson (B. campestris)

An interspecific cross between black-seeded *B. alboglabra* (CC) (Accession No. 381053) and yellow-seeded Yellow Sarson (*B. campestris*) (AA) (Accession No. 381049) was carried out to resynthesise *B. napus* (AACC) (Figure 3). As the reciprocal crosses failed to produce any viable hybrid seeds *in vivo*, the application of an embryo rescue technique (described below) followed by *in vitro* culture was used to obtain hybrid plants.

Step III resulted in the black-seeded *B. napus* line No. 01.

### Step IV: Generation of Yellow-seeded B. napus

### (Yellowish-Brown-seeded B. napus (No. 06) x Black-seeded resynthesized B. napus (No. 01))

A conventional crossing were made between the lines No. 06 and No. 01 (Figure 3) and pedigree breeding was followed. In the F₆ generation, complete yellow seeded lines were obtained.

Step IV resulted in yellow-seeded *B. napus* lines 13-217 and 13-219.

### Step V: Development of Yellow-seeded B. napus of double low quality (zero erucic acid in seed oil and low glucosinolate(s) in seed meal)

In order to develop a yellow-seeded *B.napus* line of double low quality (zero-erucic acid and low glucosinolate(s)), yellow-seeded *B. napus* lines (13-217 and 13-219) were crossed with conventional *B. napus* varieties of double low quality such as Polo and Dakini. A microspore culture technique (Lichter 1989) was applied on all resulting F₁ plants to generate doubled haploid (DH) lines. One zero erucic acid yellow-seeded DH line was crossed with the two (Polo and Dakini) conventional *B. napus* varieties of double low quality to develop yellow-seeded *B. napus* of double low quality. Pedigree breeding was followed for this purpose.

### Step VI: Generation of trigenomic hexaploids

### (Yellow-seeded B. carinata x yellow-seeded Yellow Sarson (B. campestris)

In order to assess the joint action of the transparent seed coat genes of *B. carinata* (BBCC) and Yellow Sarson *B. campestris* (AA) on seed coat pigmentation, fertile trigenomic hexaploids (AABBCC) were generated by chromosone doubling of trigenomic haploids (ABC) from {yellow-seeded *B. carinata* x yellow-seeded Yellow Sarson (*B. campestris*)} plants of Step I.

### Embryo Rescue Technique

The steps for the embyo rescue technique were as follows:
(i) the siliquae were harvested at the correct stage of development;
(ii) the green embryos (survived embryos) were rescued and were cultured *in vitro;* and
(iii) the survived embryos were sub-cultured for shoot regeneration.

In a particular detailed embodiment, *B. alboglabra* plants were cross pollinated with pollen from Yellow Sarson (*B. campestris*) and immature siliquae at 24 to 28 days after pollination were harvested, excised and the hybrid embryos were rescued using the following sterile and cell culture procedures. Excised siliquae were surface sterilised with 70% ethanol for three minutes followed by treatment with a 5% calcium hypochloride solution for 20 minutes. Siliquae were washed three times with sterile water and logitudinally dissected under a stereo microscope. The developed (fertilised) ovules were excised and obtainable embryos were rescued. These rescued embryos were cultured at 24°C in Murashige and Skoog's (1962) medium supplemented with 0.1 mg NAA and 0.1 mg Kinetin per litre of medium and under a continuous illumination of 800 lux. After 20-30 days of culture, survived embryos were sub-cultured in the same medium for shoot induction.

### Chromosome Doubling

(a) Chromosome doubling of *in vitro* culture regenerated shoots
   The essential steps for the chromosome doubling technique were as follows:
   (i) the shoots were excised and were submerged in an aqueous solution of colchicine;
   (ii) after treatment with colchicine, the shoots were washed with sterile water and the lower part of the stem was removed by cutting; and
   (iii) the shoots were transferred into rooting media.

   In a particular detailed embodiment, chromosome doubled plants were obtained by treating regenerated shoots at 2-4 leaf stage (unfolded leaf) with an aqueous solution of 0.5% colchicine plus 2.5% DMSO (modified version of Gland (1981). Shoots were submerged in this solution up to the level of the meristem/shoot apex. After 16-20 hours, shoots were taken out, washed with sterile water for three times and the 3-5mm lower parts of the shoots were cut off. Shoots were then transferred into Murashige and Skoog's (1962) medium supplemented with 3 mg IBA per litre of medium for root induction and were placed under a continuous illumination of 800 lux.
(b) Chromosome doubling of plant branches *in vivo*
   The essential steps for the chromosome doubling technique were as follows:
   (i) the leaf exils was injected with an aqueous solution of colchicine;
   (ii) repeat injection were administered in the same place at intervals.

In a particular detailed embodiment, chromosome doubled trigenomic hexaploid branches/shoots were obtained by injecting trigenomic haploids (ABC) with an aqueous solution of 0.5% colchicine plus 2.5% DMSO (modified version of Gland 1981). Specifically, 38 leaf axils from 19 plants were injected three times with the colchicine/DMSO solution at 24 hour intervals. The hexaploid nature of the branches was confirmed by visual observation as well as by flow-cytometric analysis of nuclear DNA. These branches produced fertile flowers, that is, they had viable pollen and on selfing produced viable seeds, in contrast to the flowers of the other branches (trigenomic haploid) which were sterile.

### Flow Cytometric Analysis

Flow-cytometric analysis was carried out following the methods described by Galbraith *et al.* (1983) and Sabharwal and Dolezel (1993). For this purpose, nuclear DNA from seedlings raised from selfed seeds were used.

### Isozyme Electrophoresis

Leaves (approximately 3 cm in size) from three-week-old plants were homogenised in 0.1M Tris-HC1 buffer (pH 7.2) containing 0.5% DDT (1-4 Dithio-DL-threitol) and centrifuged at 20,000 rpm for 1.5 minutes after which the supernatants of the samples were adsorbed on 9 mm x 3 mm filter paper (Whatman No. 1). A horizontal starch gel (13% starch hydrolysed, Sigma S-4501) was prepared and the samples were applied to cover approximately 1/3 of the gel-width from the cathodal end of the gel. The gel and tray buffer systems were 5 mM L-Histidine-mono-hydrochloride (adjusted with NaOH to pH 7.0) and 0.4 M tri-sodium-citrate-di-hydrate 0.1 M citric acid, (pH 7.0) respectively. The electrophoresis was carried out for 3.5 hours at 200 V, 150 mA at 0°C to ensure an adequate separation of the enzymes. All subsequent staining procedures were carried out according to Murphy et al. (1990).

### Erucic acid measurement

Erucic acid content in seed oil was measured by gas chromatographic analysis following the method described by Association of Official Analytical Chemists (1990). Typically, seed oil (triglyceride of fatty acids) was extracted from ground seeds with hexane and methylated by addition of methanol. The methylesters of fatty acids were analysed by gas chromatography using a HP5890 Series II instrument supplied with a FID detector and autosampler coupled with ChemStation HP3365. The column was a 50 m long and 0.32 mm wide WCOT fused silica (Chrompack) with a stationary of CP-sil-58CB. Helium was employed as carrier gas at a flow rate of 276 ml/min. An oven temperature of 205°C, an injection temperature of 250°C and a detection temperature of 270°C were used.

### Seed Glucosinolate(s) (GLS) measurement

A quantitative measurement of total seed glucosinolate(s) (GLS) was carried out using the method described by Smith *et al.* (1985). A qualitative measurement of seed GLS levels, using the Glucose-Test method, was carried out as follows: Two hundred microlitres of distilled water was added to five crushed seeds and incubated at room temperature for ten minutes. A Medi-Test Glucose strip (manufactured by Macherey-Nagel) was inserted into the solution and the colour change which appeared after thirty to sixty seconds was rated on a scale of one to five.

### Chemical fingerprinting of transparent seed coats compared to black seed coats of B. napus

Seed coats were analysed essentially according to the method of Andreasen *et al.* 1998 (Journal of the Science of Food and Agriculture, in press) for the extraction of soluble and insoluble esterbound phenolic acids. Seed coats of line 11A5156.082-K1217 (transparent seed coat) (Figure 6B) and the variety Miro (black seed coat) (Figure 6A) were prepared manually and further purified by a gravimetrically method employing a vertical air stream. The seed coats (100 mg) were homogenised using a mortar, added 100 ml 1.0 M NaOH and incubated at room temperature for 18 hours under N₂ in the dark. Then the pH of the hydolysates was adjusted to below 2 with 1.0 M HC1, extracted 3 times with 40 ml ethylacetate. The combined ethylacetate fractions were evaporated under vacuum at room temperature and the dried residue was dissolved in 3.0 ml of 50% acetonitrile and 2.0% trifuoroacetic acid and filtered through a 0.45 µm nylon filter.

The extracts were analysed by HPLC (Hitachi, Merck) equipped with an analytical RP-18 LiChroCART250-4 (5 µm) column at 35°C using a gradient solvent system consisting of solvent A (50% acetonitrile and 0.5% trifluoroacetic acid) and Solvent B (8% acetonitrile and 0.5% trifluoroacetic acid) with the following elution profile: 0-10 min: 100% B, 11-20 min: 90% B, 21-30 min: 85% B, 31-45 min: 80%, 46-50 min: 75% B, 51-65 min: 100% A and 66-80 min: 100% B. Flow rate: 1 ml/min. Injection volume: 50 µl. Detection with a photodiodearray detector at 280 nm and 360 nm.

### Image Analysis

Six different seed samples were used for this purpose:

| Sample number | Seed colour |
|---|---|
| 1 | Black |
| 2 | Yellow |
| 3 | Yellow |
| 4 | Black |
| 5 | Yellow |
| 6 | Dark Brown |

Image analysis was done with Agrovision Grain Check where reflection of red, green and blue colour light as well as total light reflection was measured. Data were analysed using multivariate analysis system, where only Principal Component analysis (PCA) was done.

### RESULTS

### Step I

### Interspecific cross {B. carinata x Yellow Sarson (B. campestris)}

When the *B. carinata* line was used as the female in the interspecific cross, 3.28 hybrid seeds per pollination were obtained. The reciprocal cross yielded 5.15 seeds per pollination. Although the degree of interspecific crossability between these two species was adequate, a greater number of hybrids (approximately 66%) were obtained by the application of an embryo rescue technique. Thus, the use of the embryo rescue technique for this purpose is a highly preferred feature of the present invention.

### Morphology of the trigenomic (ABC) F₁ hybrid plants

The trigenomic F₁ hybrids were easily distinguished from their parental lines by their morphological characteristics. For example, the leaf hairiness, leaf base clasping and the yellow petal colour of the F₁ hybrids was comparable to the *B. carinata* line whereas the leaf margin dentation was comparable to that of Yellow Sarson (*B. campestris*). The confirmation of these interspecific hybrids was carried out using the isozyme electrophoretic procedure as already described.

### Step II

### Crossability of the trigenomic F₁ hybrids (ABC) with B. napus (AACC)

Haploid genome composition of the trigenomic hybrids conferred very low fertility. On crossing with conventional *B.napus*, the hybrid plants produced approximately 0.025 hybrid seeds per pollination. Almost the same number of fertilised ovules were aborted before reaching to full maturity.

### Fertility of the hybrids derived from the ABC x AACC interspecific cross

The pollen fertility of the hybrids, generated from crossing the trigenomic haploids (ABC) with natural *B. napus* (AACC), was only 16.6%. As a result, the manual selfing of individual flowers, whereby pollen from fully developed flowers was placed on the stigma, was necessary to harvest a sufficient number of F₂ seeds. All harvested seeds had a black seed coat.

### Selection for seed coat colour from the ABC x AACC (F₂ to F₇ generations)

Two hundred and seventy two F₂ plants were raised (Table 1). The pollen fertility in these plants varied from 0% to 79%. In order to avoid any cross-pollination, the floral raceme of all of the F₂ plants were isolated individually by parchment paper bags. One hundred and six (39%) of the F₂ plants produced seeds using this bag isolation procedure. Of these seed bearing F₂ plants, 97 had a black seed coat while 9 had a brown seed coat. One hundred and sixteen F₃ plants were raised from the 9 F₂ brown seed families of which 99 plants produced viable seeds under bag isolation. One single F₃ family (No. 0026.002) segregated for black, brown and light brown seed colour while the other 8 families produced only black/brown coloured seeds. The light brown coloured F₄ seeds produced on 2 F₃ plants were used for further selection but an improvement in seed coat colour, in terms of obtaining a yellowish brown seed, was only obtained from the progeny of 1 (No. 0026.092) of the 2 F₃ plants. The progeny of the yellowish brown coloured seed could not be completely stabilised in the following F5 and F6 generations. However, the yellowish-brown-seed coloured F₇ line (No. 06) was crossed with the resynthesised *B. napus* (resynthesised from *B. alboglabra* x Yellow Sarson (*B. campestris*) line (No. 01) with a view to developing a true breeding yellow-seeded *B. napus* plant.

### Step III: Interspecific cross to resynthesise B. napus

### (Black-seeded B. alboglabra x Yellow-seeded Yellow Sarson (B. campestris)

An interspecific cross between black-seeded *B. alboglabra* (CC) and yellow-seeded Yellow Sarson (*B. campestris*) (AA) was carried out to resynthesise *B. napus* (AACC) (Figure 3). As the reciprocal crosses failed to produce any viable hybrid seeds *in vivo*, an embryo rescue technique (described above) was applied followed by *in vitro* culture. Thus, the use of the embryo rescue technique for the resynthesis of *B. napus* is regarded as an essential technical feature of the present invention. For this purpose, *B. alboglabra* was cross pollinated with Yellow Sarson (*B. campestris*) and approximately 0.45 embryos per cross pollinated siliquae were rescued of which 40% survived under *in vitro* culture conditions and gave rise to amphihaploid (AC) plantlets. The plantlets were treated with aqueous solution containing 0.2% colchicine plus 2.5% DMSO for doubling chromosomes. The chromosome doubled resynthesized *B. napus* was self-compatible in nature and had the white petal characteristic of the *B. alboglabra* line and on selfing produced black coated seeds.

### Step IV: Generation of completely yellow-seeded B. Napus

### (Yellowish-brown-seeded B. napus (No. 06) x Black-seeded resynthesized B. napus (No. 01))

A conventional crossing were made between the lines No.06 and No.01. The low (approximately 10 seeds) yield of seeds per pollination (Table 2) was expected as both of the parental lines were developed through the interspecific crosses already described.

Of the selfed seeds of 259 F₂ plants, which were visually examined for colour (Table 3), 199 (76.8%) produced black/brown coat coloured seeds, 51 (19.7%) produced partly-yellow coloured seeds and 9 (3.5%) produced yellow-brown coloured seeds. F₃ families were generated from the selfed seeds of all the 9 yellow-brown seeded F₂ plants. Of the 9 F₃ families, 4 families segregated for black/brown and partly yellow seeds in a proportion of 19:41, 4 families segregated for black/brown, partly-yellow and yellow-brown seeds in a proportion of 19:48:62 and 1 F₃ family segregated for partly-yellow, yellow-brown and nearly yellow seeds in a proportion of 9:18:1. Thus, 1 nearly yellow-seeded F₃ plant was obtained from a total of 217 F₃ plants from 9 families. Selfed seeds of the single nearly yellow-seeded and selected 8 yellow-brown seeded F₃ plants were used to grow F₄ families. No yellow-seeded plant was found in the F₄ families which were generated from the yellow-brown seeded F₃ plants. On the other hand, the F₄ family generated from the nearly yellow-seeded F₃ plant segregated again for partly-yellow, yellow-brown and yellow seeds. The proportion of yellow seeded plants (40.9%) in this generation was significantly higher than that obtained in the F₃ generation. The F₅ and F₆ generation families were grown only from the selected yellow-seeded plants. Segregation for yellow-brown and yellow seed colour continued with dominance of the yellow-seeded plants in each successive generation. All of the seeds obtained in the F₇ generation had a consistent and stable yellow colour.

Because the yellow-seeded lines thus developed are derived from a series of interspecific crosses, a low fertility in these plants could be expected. Selection for improved fertility was performed along with selection for a consistent and stable yellow seed colour as follows: The yellow-seeded F₄ to F₉ generation plants were crossed with natural *B. napus* using the yellow-seeded plants as the female. The extent of crossability was increased in each successive generation from 3 seeds per pollination in the F₄ generation almost 7 seeds per pollination in the F₉ generation (Table 4).

The seed oil and seed meal of the yellow-seeded *B. napus* obtained in the present case contained an intermediate level of erucic fatty acid (26 - 28%) and a high level of glucosinolate(s) (>40µmol/g seed), respectively. Such two lines (13-217 and 13-219) were crossed with conventional oilseed rape *B. napus* of double low quality in order to develop yellow seeded oilseed rape *B. napus* of double low quality. The above-mentioned two yellow-seeded *B. napus* lines had white petal, apparently derived from the C-genome of *B. alboglabra.*

### Step V: Development of yellow-seeded oilseed rape B. napus of double low quality (zero erucic acid in oil and low glucosinolate(s) in seed meal)

F₁ hybrids from crosses between yellow seeded lines (13-217 and 13-219) and conventional *B. napus* of double low quality such as cultivars Polo and Dakini were used to produce doubled haploid (DH) plants/lines. Two hundred and eighty seven DH plants/lines were generated from four crosses (Table 5).

### Inheritance of seed coat colour in doubled haploid (DH) population

The following segregation pattern for seed colour was found in 287 DH lines (Table 5): 195 DH lines had black/dark-brown-seed colour; 34 were reddish-brown, 27 were partly-yellow, 14 were yellow-brown and 17 yellow-seeded. When the data from the black/dark brown, reddish brown, partly yellow and yellow-brown seed coloured DH lines were pooled into one class, a 270:17 distribution pattern was obtained which fits in well with a 15:1 segregation pattern for four gene loci. In contrast, when the data from the black/dark brown, reddish brown and partly yellow seed coloured DH lines were pooled into one class and the data from the yellow-brown and yellow seed coloured DH lines were pooled into a second class, a distribution pattern of 256:31 which fits with a 7:1 segregation pattern for three gene pairs was obtained. It would only be logical to pool the data from the latter two classes (yellow and yellow-brown classes) if the parental yellow-seeded lines used in the crosses would in any instance produce yellow-brown seeds. Selfing 30 plants of these two parental yellow-seed coloured lines grown in the field indicated an instability for seed coat colour in 30% of the plants examined. However, such instability was not found when the plants were grown in glasshouse. Although the chi-square values obtained were not significant for both the four and the three-gene loci model, a better fit to the segregation ratio (in terms of chi-square and p-values) was found with a four-gene loci model indicating that the transparent seed coat colour for yellow seed is due to a homozygous recessive condition in all four loci. As the seed coat colour in *B. campestris* is governed by two loci and the transparent seed coat colour (yellow seed) is due to homozygous recessive condition in both loci (Stringam 1980, Schwetka 1982), the results suggest that a four gene model for the transparent seed coat colour in *B. napus* is the most appropriate to consider.

### Inheritance of petal colour and erucic acid content in doubled haploid (DH) lines

A total of 61 doubled haploid lines from four crosses were examined for petal colour and erucic acid content (Table 6). Twenty six (26) lines had a yellow petal colour and thirty five (35) lines had a white petal colour. This 26:35 distribution ratio agrees with a 1:1 Mendelian segregation pattern (X²=1.05, p=0.5-0.3). Seeds of 27 DH lines were almost free from erucic acid (range of 0.04 - 3.1%, mean of 0.4%) while 34 lines had an erucic acid content ranging from 15.8-33.4% (mean of 22.7%). This 27:34 distribution ratio is in accordance with a 1:1 Mendelian segregation (X²=0.59, p=0.5-0.3) indicating that a single pair of functional alleles for erucic acid content was present in the two yellow-seeded lines. All of the white petal flowered lines were associated with the presence of erucic acid, except for two lines (0.5 and 3.1% erucic acid) which were almost free from erucic acid. Similarly, all yellow-flowered lines were associated with the absence of erucic acid, except for one line which contained 25.3% erucic acid. These results suggests that the gene locus controlling the petal colour and the locus controlling erucic acid content in the C-genome are located on the same chromosome but that recombination between these two loci can sometimes occur, which in present case, was in 4.9% of the DH lines.

### Inheritance of total glucosinolate(s) (GLS) content in doubled haploid (DH) lines

Using the method of Smith *et al* (1985), seeds of a total of 202 DH lines from the four crosses were examined for GLS content (Table 7). Seeds from 7 lines had less than 20 µmol GLS per g seed while seeds from the remaining 195 lines had greater than 20 µmol GLS per g seed. This 7:195 distribution clearly agrees with the 1:15 segregation pattern for four gene loci. The segregation pattern in each individual cross did not depart significantly from the overall pattern.

From the above mentioned doubled haploid breeding programme, it was possible to obtain a yellow seed coloured *B. napus* line (154.004) whose seed is free from erucic fatty acid and whose seed meal contained a high level of GLS. In order to lower the seed GLS level of the yellow-seeded *B. napus*, the DH line (154.004) was crossed with conventional oilseed rape of double low quality such as Polo and Dakini. F₂ populations were raised in field plots. Seeds of a total of 144,914 open pollinated F₂ plants were examined for seed coat colour from which open pollinated seeds of 640 plants were harvested as being yellow-seeded or nearly yellow seeded.

The Glucose-Test method was applied for qualitative measurement of GLS content in seeds of these 640 yellow-seeded plants. Using the Glucose Test results, seeds of 507 plants which displayed a very high test score (3-5), were deemed as having a high GLS level and were discarded. A quantitative GLS measurement was carried out on the seeds from the remaining 133 plants that had a test score of 2. Seeds from 12 plants, having relatively low GLS content (25-40 µmol/g seed), were used to develop F₃ generation plants. A total of 600 F₃ plants were grown, selfed and examined for seed colour. Two hundred eighty seven plants produced yellow or yellow-brown or partly-yellow coloured seeds while the remaining 313 plants produced brown or black coloured seeds. The Glucose-Test was carried out on seeds from the above-mentioned 287 plants from which 87 yellow, 24 yellow-brown and 23 partly-yellow seed coloured families were selected for developing F₄ generation plants. Of the 87 F₄ families descended from the yellow-seed coloured F₃ plants, 40 families were stable for yellow seed colour, while 47 F₄ families again segregated for yellow and yellow-brown seed colour. As expected, segregation for seed colour was also observed in the progenies of 24 yellow-brown- and 23 partly-yellow-seed coloured F₃ plants. Selfed seeds from a total of 402 F₄ plants were examined for seed colour and 114 were discarded due to brown seed. Quantitative measurement of GLS was carried out on selfed seeds from the remaining 288 F₄ plants (yellow + yellow-brown + partly-yellow seed). Seeds from five plants were shown to have a GLS level less than 20 µmol/g seed and were yellow in colour.

These results indicate that yellow-seed *B. napus* may be developed for commercialisation with varying levels of seed oil and seed meal quality such as: (i) zero erucic acid and low glucosinolate(s), (ii) zero erucic acid and high glucosinolate(s), (iii) high erucic acid and low glucosinolate(s), (iv) high erucic and high glucosinolate(s).

### Step VI: Synthesis of the trigenomic hexaploid (AABBCC) from the B. carinata x Yellow Sarson (B. campestris) interspecific cross and its seed coat colour

Trigenomic hexaploids were obtained by injecting trigenomic haploids (ABC) with an aqueous solution of 2.5% DMSO plus 0.2% colchicine which induces chromosome doubling (Figure 2). The 38 leaf axils from 19 plants which were injected with the colchicine/DMSO solution yielded 18% fertile trigenomic hexaploid shoots (branches) which produced 5.4 to 35.0% viable pollen and were self-compatible. Using manual selfing, an approximately 93% siliqua set was obtained and the number of viable seeds harvested per selfing was 3.27. All selfed seeds were uniform brown in colour. The next generation of plants were grown from selfed seeds and the hexaploid nature of the plants were confirmed by flow-cytometric analysis of nuclear DNA. Selfed seeds produced on these plants were also brown.

### Chemical fingerprinting of transparent seed coats compared to black seed coats of B. napus

In order to establish whether the transparent appearance of the seed coats is due to a reduction of the concentration of chromogenic substances compared to black seed coats, chemical fingerprinting on HPLC of alkaline seed coat hydrolysates were conducted (Figures 4-5).

The chromatogrammes showed that some light absorbing substances were present in much lower quantities in transparent seed coats compared to black seed coats. For example, a substance eluting with a retention time of 7.50-7.53 min was present in a 35-fold lower concentration in the transparent seed coats than in the black seed coats, measured at 280 nm (Figures 4A-5A). On average, the transparent seed coats contained 4.6-fold lower concentrations of substances absorbing at 280 nm.

Similar data were obtained by measuring the absorbance at 360 nm simultaneously (Figures 4B-5B). For example, two compounds eluting at 17.04-17.11 and 26.78-26.82 min were found in about 8-fold lower concentrations in the transparent seed coats compared to the black seed coats, respectively. As these two compounds displayed significant absorption at both 280 nm and 360 nm, it is possible that they are flavonoids as the group of substances with this characteristic absorption are often associated with colorization of plant tissues and are preferentially extracted by the employed analytical method (Norbaek *et al.* 1998. Phytochemistry, in press).

### Image Analysis

PCA-plot of Agrovision colour data on the 6 seed samples is shown in Figure 7, where the first principal component (x-axis) is for colour intensity which explained 91% of the recorded total variation among the 6 seed samples. The present data clearly distinguish the yellow seeds samples (No. 2, 3 and 5) from the remaining black (No. 1 and 4) and brown (No. 6) seeds samples. Within the dark seed samples slight variation was found while no variation exists between the three yellow seed samples.

### Oil, protein and fibre content in yellow-seeded B. napus

### Materials and Methods

Two yellow-seeded F₇ lines, 13-217 and 13-219, were crossed with two black-seeded spring type *B. napus* cvs. 'Polo' and 'Dakini'. Microspore culture technique (Lichter 1989) was applied for production of DH lines from F₁ plants. Bulk seed samples of black, brown and yellow coloured seeds were prepared using a small amount of seeds from these three seed colour type DH lines and were used for measurement of oil, protein and fibre content. Oil and protein content was measured by near infrared reflectance, using a suitably calibrated InfraAlyzer2000 (Bran + Luebbe) and fibre content was measured following the method described by Stringam et al. (1974).

### Results

Oil, protein and fibre data from bulk seed samples of black-, brown- and yellow-seeded DH lines are presented in Table 8. The yellow seed sample had higher oil + protein contents

**Table 8: Oil, protein and fibre content (in bracket, relative values) in different coloured bulk seed samples of doubled haploid lines from yellow-seeded x black-seeded B. napus cross.**

| Seed colour | Oil + protein | | Fibre | | Fibre | |
|---|---|---|---|---|---|---|
| | % seed DM* | | % seed | | % oil-free meal | |
| Black seed | 68.2 | (100) | 8.6 | (100) | 13.6 | (100) |
| Brown seed | 69.8 | (102) | 5.9 | (69) | 9.6 | (71) |
| Yellow seed | 70.4 | (103) | 3.9 | (45) | 6.1 | (45) |

| | | | | | | |
|---|---|---|---|---|---|---|
| *DM = dry matter | | | | | | |

than its black-seeded counterpart. Oil and protein content are negatively correlated to each other (Grami et al. 1977, Bengtsson 1985). This indicates that oil or protein or both oil and protein content in yellow-seeded *B. napus* can be increased to a level higher than black-seeded types through further breeding. Fibre content in yellow seed as well as in oil-free meal from yellow seed was reduced by about 55% compared to black seed or oil-free meal from black seed.

### DISCUSSION

### Source of the transparent seed coat gene in the CC genome of yellow-seeded B. napus derived from the No. 06 x No. 01 cross

The seed coat colour of the parent *B. napus* line No. 01 (AACC) which was resynthesized from the black-seeded *B. alboglabra* (CC) x Yellow Sarson (*B. campestris*) (AA) cross is completely black. This line carries the transparent seed coat gene in its AA genome and lacks the transparent seed coat gene in its CC genome. Thus, the generation of a completely yellow-seeded *B. napus* from a No. 06 x No. 01 cross is not possible without a transparent seed coat gene in the CC genome of the No. 06 line.

The transparent seed coat gene in the CC genome of the *B. napus* line No. 06 is the transparent seed coat gene of Yellow Sarson (*B. campestris*) which has been transferred through allosyndesis between the A- and the C-genome chromosomes during development of the line No. 06 from the interspecific cross.

The possibility that the transparent seed coat gene in the CC genome of the No.06 was directly derived from the *B. carinata* (BBCC) line was eliminated by the demonstration that the synthesis of trigenomic hexaploids (AABBCC) from the yellow-seeded *B. carinata* x Yellow Sarson (*B. campestris*) crosses failed to produce a transparent seed coat colour, that is, yellow seeds.

Furthermore, from a separate study, the partly yellow-seeded CC-genomic species, *B. alboglabra* was resynthesised from the {(yellow-seeded *B. carinata* x black-seeded *B. alboglabra*) x black-seeded *B. alboglabra*} cross. Using this partly yellow-seeded *B. alboglabra* (CC) and yellow-seeded *B. campestris* Yellow Sarson (AA), *B. napus* (AACC) was resynthesised. The resynthesised *B. napus* was completely black-seeded. A similiar approach by Chen (Chen *et al* 1988; Chen and Heneen 1992) failed to produce any yellow-seeded *B. napus*.

These results clearly demonstrate that: (i) the parent, F₇ generation, yellowish brown-seeded *B.napus* line (No.06), developed from the {{*B. carinata* x Yellow Sarson *(B. campestris)}* x *B. napus*} cross carries the gene for transparent seed coat colour of Yellow Sarson (*B. campestris*) in its CC genome and (ii) the transparent seed coat gene of *B. carinata* in combination with the transparent seed coat gene of Yellow Sarson (*B. campestris*) fails to produce yellow-seeded *B. napus*.

### SUMMATION

The present invention therefore provides transformed plants comprising a transparent seed coat gene.

In a preferred aspect the present invention describes the development of stable and consistent transparent-seed coat coloured i.e. yellow-seeded oilseed rape *B. napus* from interspecific crosses. In this respect, the transparent seed coat gene of the AA genome of Yellow Sarson (*B. campestris*) were transferred into the CC genome of *B napus* through allosyndesis between the A- and the C-genome chromosomes. The resultant *B napus* AACC genome, carrying the transparent seed coat gene of Yellow Sarson (*B. campestris*), yielded a consistent and stable yellow seed coloured *B. napus* plant line.

As will be apparent - particularly from the teachings of the Examples - the application of the present invention is not technically confined to a single plant variety.

Various modifications and variations of the described aspects of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in plant biology and/or plant biotechnology and/or plant molecular biology and/or related fields are intended to be within the scope of the following claims.

### TABLES

**Table 1. Selection for yellow-seeded B. napus from interspecific cross derived trigenomic haploids (ABC) x B. napus (AACC) cross**

| Parentage | Mother Plant | | Examined Generation | Number plants grown | Per cent plants producing seeds | Number of plants for seed colour | | | |
|---|---|---|---|---|---|---|---|---|---|
| | Generation | Seed colour | | | | Black | Brown | Light brown | Yellow ish brown |
| (Cari x Cam) x Nap | F₁ | Black | F₂ | 272 | 39.0 | 97 | 9 | - | - |
| (Cari x Cam) x Nap | F₂ | Brown | F₃ | 116 | 85.3 | 82 | 15 | 2 | - |
| (Cari x Cam) x Nap | F₃ | Light brown | F₄ | 50 | 82.0 | - | 7 | 28 | 6 |
| (Cari x Cam) x Nap | F₄ | Yellowish | F₅ | 20 | 70.0 | - | - | 4 | 10 |
| | | brown | | | | | | | |
| (Cari x Cam) x Nap | F₅ | Yellowish | F₆ | 40 | 92.5 | - | 10 | 12 | 15 |
| | | brown | | | | | | | |
| (Cari x Cam) x Nap | F₆ | Yellowish | F₇ | 20 | 100 | - | - | 7 | 13 |
| | | brown | | | | | | | |

| | | | | | | | | | |
|---|---|---|---|---|---|---|---|---|---|
| Cari = *B. carinata* (Yellow Seed), 381078, Cam = *B*. *campestris* Yellow Sarson (Yellow Seed), 3-0166.001, Nap = *B*. *napus* (Black Seed), 1-9007 | | | | | | | | | |

**Table 2. Crossability between interspecific cross derived resynthesized B. napus lines No.06 and No.01 and natural B. napus cv. Jaguar.**

| Cross | Number pollination | Number seeds per pollination |
|---|---|---|
| No.06 x No.01 | 17 | 9.4 |
| No.01 x No.06 | 10 | 10.3 |
| No.06 x Jaguar | 28 | 16.9 |
| Jaguar x No.06 | 10 | 26.1 |
| No.01 x Jaguar | 15 | 9.8 |
| Jaguar x No.01 | 31 | 18.5 |

**Table 3. Selection for yellow seed in the progenies generated from crossing two resynthesized B. napus lines, No.06 x No.01.**

| Mother plant | | Examined plant generation | Number families grown | Total number plants | Black/brown seed | | Partly yellow seed | | Yellow brown seed | | Yellow seed | |
|---|---|---|---|---|---|---|---|---|---|---|---|---|
| | | | | | | | | | | | | |
| | | | | | | | | | | | | |
| Generation | Seed colour | | | | No. | % | No. | % | No. | % | No. | % |
| F1 | - | F2 | - | 259 | 199 | 76.8 | 51 | 19.7 | 9 | 3.5 | - | - |
| F2 | Yellow | F3 | 9 | 217 | 38 | 17.5 | 98 | 45.2 | 80 | 36.9 | 1 | 0.5 |
| | brown | | | | | | | | | | | |
| F3 | Yellow | F4 | 1 | 22 | - | - | 1 | 4.5 | 12 | 54.5 | 9 | 40.9 |
| | Yellow | F4 | 8 | 103 | 17 | 16.5 | 63 | 61.2 | 23 | 22.3 | - | - |
| | brown | | | | | | | | | | | |
| F4 | Yellow | F5 | 4 | 55 | - | - | - | - | 12 | 21.8 | 43 | 78.2 |
| F5 | Yellow | F6 | 14 | 122 | - | - | - | - | 5 | 4.1 | 117 | 95.9 |
| F6 | Yellow | F7 | 2 | 25 | - | - | - | - | - | - | 25 | 100.0 |

**Table 4. Crossability of yellow seeded B. napus as female with natural B. napus.**

| Generation of yellow seeded family crossed | Number yellow seeded family used | Number pollinations | Number seeds per pollination |
|---|---|---|---|
| F4 | 1 | 30 | 3.0 |
| F6 | 6 | 208 | 4.4 |
| F7 | 2 | 55 | 4.8 |
| F8 | 2 | 70 | 6.8 |
| F9 | 3 | 55 | 6.9 |

**Table 5. Segregation for seed colour in doubled haploid (DH) lines from cross between yellow-seeded B. napus (13-217 and 13-219) and black-seeded natural B. napus (Polo and Dakini).**

| Cross | Total DH lines | Black/ dark brown seed | Reddish brown seed | Partly yellow seed | Yellow brown seed | Yellow seed | Segregation 15:1^{a} | | Segregation 7:1^{b} | |
|---|---|---|---|---|---|---|---|---|---|---|
| | | | | | | | X² | p | X² | p |
| Polo x 13-217 | 75 | 52 | 8 | 8 | 3 | 4 | 0.008 | 0.9-0.95 | 0.43 | 0.5-0.7 |
| Dakini x 13-217 | 70 | 43 | 13 | 7 | 4 | 3 | 0.19 | 0.5-0.7 | 0.20 | 0.5-0.7 |
| Polo x 13-219 | 75 | 52 | 5 | 7 | 4 | 7 | 0.75 | 0.3-0.5 | 0.15 | 0.5-0.7 |
| Dakini x 13-219 | 67 | 48 | 8 | 5 | 3 | 3 | 0.12 | 0.7-0.9 | 0.48 | 0.3-0.5 |
| Total | 287 | 195 | 34 | 27 | 14 | 17 | 0.01 | 0.9-0.95 | 0.61 | 0.3-0.5 |

| | | | | | | | | | | |
|---|---|---|---|---|---|---|---|---|---|---|
| ^{a}Segregation tested after pooling data of black/dark brown, reddish brown, partly yellow and yellow-brown seed into one class and yellow seed into the other class ^{b}Segregation tested after pooling data of black/dark brown, reddish brown and partly yellow seed into one class, and yellow-brown and yellow seed into the other class | | | | | | | | | | |

**Table 6. Segregation for petal colour and erucic acid (C_{22:1}) content in doubled haploid (DH) lines from cross between yellow-seeded B. napus (13-217 and 13-219) and natural B. napus(Polo and Dakini).**

| | Yellow petal | | White petal | |
|---|---|---|---|---|
| | - C_{22:1} | + C_{22:1} | - C_{22:1} | + C_{22:1} |
| Polo x 13-217 | 3 | - | - | 7 |
| Dakini x 13-217 | 10 | - | 1 | 16 |
| Polo x 13-219 | 8 | - | 1 | 3 |
| Dakini x 13-219 | 4 | 1 | - | 7 |
| Total | 25 | 1 | 2 | 33 |

| | | | | |
|---|---|---|---|---|
| The absence (3.1% or less) and presence (15.8-33.4%) of erucic acid content is indicated by (-) and (+) signs respectively. | | | | |

**Table 7. Segregation for glucosinolate(s) (GLS) (µmol/g seed) content in doubled haploid lines from cross between yellow-seeded B. napus (13-217 and 13-219) and natural B. napus(Polo and Dakini).**

| Cross | <20 µmol GLS | >20 µmol GLS | Segregation, 1:15 | |
|---|---|---|---|---|
| | | | X² | p |
| Polo x 13-217 | 3 | 51 | 0.005 | 0.9-0.95 |
| Dakini x 13-217 | 2 | 55 | 0.34 | 0.5-0.7 |
| Polo x 13-219 | 1 | 44 | 0.65 | 0.3-0.5 |
| Dakini x 13-219 | 1 | 45 | 0.70 | 0.3-0.5 |
| Total | 7 | 195 | 2.22 | 0.1-0.2 |

### REFERENCES

Anjou K, Lönnerdal B, Uppström B, Åman P. 1977. Composition of seeds from some *Brassica* cultivars. Swedish J. Agric. Res. 7:169-178.
Association of Official Analytical Chemists, 1990. Official methods of analysis of the Association of Official Analytical Chemists (ed. K. Helrich), 15^{th} edition, Volume 2, Published by Association of Official Analytical Chemists, Virginia, USA.
Attia T, Busso C, Röbbelen G. 1987. Digenomic triploids for an assessment of chromosome relationships in the cultivated diploid *Brassica* species. Genome 29:326-330.
Attia T, Röbbelen G. 1986. Cytogenetic relationship within cultivated *Brassica* analyzed in amphihaploids from the three diploid ancestors. Can J Genet Cytol 28:323-329.
Barcikowska B, Zwierzykowska E, Balicka M. 1987. On the way to yellow-seeded *Brassica napus* L. - Hybrids of *B. campestris* x *B. oleracea* and *B. oleracea* x *B. carinata* yellow seeded. Proc. 7th Int. Rapeseed Congr., Poland 2, 492-495.
Bell JM, Shires A. 1982. Composition and digestibility by pigs of hull fractions from rapeseed cultivars with yellow or brown seed coats. Can. J. Anim. Sci. 62:557-565.
Bengtsson, L., 1985: Improvement of rapeseed meal quality through breeding for high protein content. A Ph.D. Thesis. The Swedish University of Agricultural Sciences, Department of Crop Genetics and Breeding, Svalöv, Sweden.
Busso C, Attia T, Röbbelen G. 1987. Trigenomic combinations for the analysis of meiotic control in the cultivated *Brassica* species. Genome 29:331-333.
Chen BY, Heneen WK. 1992. Inheritance of seed colour in *Brassica campestris* L. and breeding for yellow-seeded *B. napus* L. Euphytica 59:157-163.
Chen BY, Heneen WK, Jönsson R. 1988. Resynthesis of *Brassica napus* L. through interspecific hybridization between *B. alboglabra* Bailey and *B. campestris* L. with special emphasis on seed colour. Plant Breed. 101:52-59.
Daun JK, DeClercq DR. 1988. Quality of yellow and dark seeds in *Brassica campestris* canola varieties Candle and Tobin. JAOCS 65:122-126.
Galbraith DW, Harkins KR, Maddox JM, Ayres NM, Sharma DP, Firoozabady E. 1983. Rapid flow cytometric analysis of the cell cycle in intact plant tissues. Science 220:1049-1051.
Gland A. 1981. Doubling chromosomes in interspecific hybrids by colchicine treatment. Eucarpia Cruciferae Newsletter 6:20-22.
Grami, B., R.J. Baker, and B.R. Stefansson, 1977: Genetics of protein and oil content in summer rape: heritability, number of effective factors, and correlations. Can. J. Plant Sci. 57, 937-943.
Hu X-J. 1988. Studies on the relationship between seedcoat colour and pigment content in different types of rapeseed cultivars. Cruciferae Newsletter 13:44-45.
Leung J, Fenton TW, Mueller MM, Clandinin DR. 1979. Condens tannins of rapeseed meal. J. Food Sci. 44:1313-1316.
Lichter R. 1989. Efficient yields of embryoids by culture of isolated microspores of different *Brassicaceae* species. Plant Breed 103:119-123.
Liu H-L. 1983. Studies on the breeding of yellow seeded *Brassica napus*. Proc. 6th Int. Rapeseed Conf., Paris. pp. 637-641.
Liu H-L, Gao Y-T. 1987. Some fundamental problems conducted from the studies on the breeding of yellow-seeded *Brassica napus* L. Proc. 7th Int. Rapeseed Congr., Poznan, Poland. pp. 476-480.
Murashige I, Skoog, F. 1962. A revised medium for rapid growth and bioassays with tobacco tissue cultures. Physiol Plant 15:473-497.
Murphy RW, Sites Jr. JW, Buth DG, Haufler CH. 1990. Isozyme electrophoresis. In: Hillis DM and Moritz C (eds): Molecular systematics, pp. 45-126. Sinaur Associates, Sunderland, USA.
Rashid A, Rakow G, Downey RK. 1994. Development of yellow seeded *Brassica napus* through interspecific crosses. Plant Breed. 112:127-134.
Sabharwal PS, Dolezel J. 1993. Interspecific hybridization in *Brassica*: Application of flow cytometry for analysis of ploidy and genome composition in hybrid plants. Biologia Plantarum 35:169-177.
Schwetka A. 1982. Inheritance of seed colour in turnip rape (*Brassica campestris* L.). Theor. Appl. Genet. 62:161-169.
Shirzadegan M, Röbbelen G. 1985. Influence of seed colour and hull proportion on quality properties of seeds in *Brassica napus* L. Fette Seifen Anstrichmittel 87:235-237.
Simbaya, J., Slominski, BA, Rakow, G, Campbell, LD, Downey, RK, Bell JM 1995. Quality characteristics of yellow-seeded *Brassica* seed meals: Protein, carbohydrates and dietary fiber components. J. Agric. Food Chem. 43: 2062-2066.
Slominski BA, Campbell LD, Guenter W. 1994. Carbohydrates and dietary fiber components of yellow-seeded and brown-seeded canola. J. Agric. Food Chem. 42:704-707.
Stringam, G.R., D.I. McGregor, and S.H. Pawlowski, 1974: Chemical and morphological characteristics associated with seedcoat color in rapeseed. Proc. 4th Int. Rapeseed Conf., Giessen, Germany, 99-108.
Smith DB, Parsons DG, Starr C. 1985. A simple and rapid method of quantitatively measuring the glucosinolate(s) concentration of rapeseed. J. agric Sci., Camb. 105:597-603.
Stringam GR 1980. Inheritance of seed color in turnip rape. Can. J. Plant Sci., 60, 331-335.
Stringam GR, McGregor DI, Pawlowski SH. 1974. Chemical and morphological characteristics associated with seedcoat color in rapeseed. Proc. 4th Int. Rapeseed Conf., Giessen, Germany. pp.99-108.
Tang ZL, Li JN, Zhang XK, Chen L, Wang R. 1997. Genetic variation of yellow-seeded rapeseed lines (*Brassica napus* L.) from different genetic sources. Plant Breed 116:471-474.
Theander O, Åman P, Miksche GE, Yasuda S. 1977. Carbohydrates, polyphenol, and lignin in seed hulls of different colors from turnip rapeseed. J. Agric. Food Chem. 25:270-273.
Van Deynze AE, Beversdorf WD, Pauls KP. 1993. Temperature effects on seed color in black- and yellow-seeded rapeseed. Can. J. Plant Sci. 73:383-387.
Van Deynze A, Pauls KP. 1994. The inheritance of seed colour and vernalization requirement in *Brassica napus* using doubled haploid populations. Euphytica 74:77-83.
Vaughan JG. 1970. Cruciferae. In: JG Vaugan (ed) The structure and utilization of oilseeds. Chapman and Hall, London, UK. pp. 49-62.
Woods DL. 1980. The association of yellow seed coat with other characteristics in mustard *B. juncea*. Cruciferae Newsletter 5:23-24.
Zaman MW. 1988. Limitations for introgression of yellow seed coat colour in *Brassica napus*. Sveriges Utsadesförenings Tidskrift, 98:157-161.

## Claims

1. A method for producing seed having an increased level of seed oil and protein and a reduced level of fibre, wherein the method comprises:
a) crossing a yellow-seeded *B. carinata* and a yellow-seeded *B. campestris* to produce a trigenomic haploid, crossing the trigenomic haploid with *B. napus* and selfing the progeny to produce an offspring *Brassica* plant;
**characterized in that** it further comprises:
b) crossing a first *Brassica* plant having a CC genome and a second *Brassica* plant having an AA genome of a yellow seeded *Brassica campestris,* applying an embryo rescue technique followed by *in vitro* culture to produce an offspring *Brassica;* and
c) crossing the offspring *Brassica* plant of step (a) with the offspring *Brassica* plant of step (b) and selfing the progeny to produce a yellow-seeded *Brassica* plant wherein said yellow-seeded *Brassica* plant produces seeds having a consistent and stable yellow colour.

2. A method for producing a *Brassica* plant, plant cell or plant tissue by selective cross-breeding wherein the method comprises:
a) crossing a yellow-seeded *B. carinata* and a yellow-seeded *B. campestris* to produce a trigenomic haploid, crossing the trigenomic haploid with *B. napus* and selfing the progeny to produce an offspring *Brassica* plant;
**characterized in that** it further comprises
b) crossing a first *Brassica* plant having a CC genome and a second *Brassica* plant having an AA genome of a yellow seeded *Brassica campestris,* applying an embryo rescue technique followed by *in vitro* culture to produce an offspring *Brassica;* and
c) crossing the offspring *Brassica* plant of step (a) with the offspring *Brassica* plant of step (b) and selfing the progeny to produce a yellow-seeded *Brassica* plant wherein said yellow-seeded *Brassica* plant produces seeds having a consistent and stable yellow colour.

## Patentansprüche

1. Verfahren zum Erzeugen von Samen mit einem erhöhten Gehalt an Samenöl und Protein und einem reduzierten Gehalt an Fasern, wobei das Verfahren folgendes umfaßt:
a) Kreuzen einer *B. carinata* mit gelben Samen und einer *B. campestris* mit gelben Samen, um ein drei Genome aufweisendes Haploid zu erzeugen, Kreuzen des drei Genome aufweisenden Haploids mit *B. napus* und Vermehren der Nachkommenschaft mit sich selbst unter Erzeugung einer *Brassica*-Nachzuchtpflanze,
**dadurch gekennzeichnet, daß** es weiterhin folgendes umfaßt:
b) Kreuzen einer ersten *Brassica*-Pflanze, die ein CC-Genom hat, und einer zweiten *Brassica-*Pflanze, die ein AA-Genom einer *Brassica campestris* mit gelben Samen hat, Anwenden einer Embryorettungstechnik, gefolgt von Kultivierung *in vitro* unter Erzeugung einer Brassica-Nachzucht und
c) Kreuzen der *Brassica*-Nachzuchtpflanze aus Stufe (a) mit der *Brassica*-Nachzuchtpflanze aus Stufe (b) und Vermehren der Nachkommenschaft mit sich selbst unter Erzeugung einer *Brassi*ca-Pflanze mit gelben Samen, wobei die *Brassica*-Pflanze mit gelben Samen Samen erzeugt, die eine konsistente und stabile gelbe Farbe haben.

2. Verfahren zum Erzeugen einer *Brassica*-Pflanze, einer -Pflanzenzelle oder von -Pflanzengewebe durch selektive Kreuzung, wobei das Verfahren folgendes umfaßt:
a) Kreuzen einer *B. carinata* mit gelben Samen und einer *B. campestris* mit gelben Samen, um ein drei Genome aufweisendes Haploid zu erzeugen, Kreuzen des drei Genome aufweisenden Haploids mit *B. napus* und Vermehren der Nachkommenschaft mit sich selbst unter Erzeugung einer *Brassica*-Nachzuchtpflanze,
**dadurch gekennzeichnet, daß** es weiterhin folgendes umfaßt:
b) Kreuzen einer ersten *Brassica*-Pflanze, die ein CC-Genom hat, und einer zweiten *Brassica-*Pflanze, die ein AA-Genom einer *Brassica campestris* mit gelben Samen hat, Anwenden einer Embryorettungstechnik, gefolgt von Kultivierung *in vitro* unter Erzeugung einer Brassica-Nachzucht und
c) Kreuzen der *Brassica*-Nachzuchtpflanze aus Stufe (a) mit der *Brassica*-Nachzuchtpflanze aus Stufe (b) und Vermehren der Nachkommenschaft mit sich selbst unter Erzeugung einer *Brassi*ca-Pflanze mit gelben Samen, wobei die *Brassica*-Pflanze mit gelben Samen Samen erzeugt, die eine konsistente und stabile gelbe Farbe haben.

## Revendications

1. Procédé pour produire des semences ayant une teneur accrue en huiles et protéines des semences et une teneur réduite en fibres, le procédé comprenant :
a) le croisement d'une plante *B. carinata* à semences jaunes et d'une plante *B. campestris* à semences jaunes pour produire un haploïde trigénomique, le croisement de l'haploïde trigénomique avec *B. napus* et l'autofécondation de la descendance pour produire un plant de *Brassica* comme descendance ;
**caractérisé en ce qu'**il comprend en outre :
b) le croisement d'un premier plant de *Brassica* ayant un génome CC et d'un second plant de *Brassica* ayant un génome AA d'un *Brassica campestris* à semences jaunes, l'application d'une technique de sauvetage d'embryon avec ensuite une culture *in vitro* pour produire un plant de *Brassica* comme descendance ; et
c) le croisement du plant de *Brassica* obtenu comme descendance de l'étape (a) avec le plant de *Brassica* obtenu comme descendance de l'étape (b) et l'autofécondation des descendants pour produire un plant de *Brassica* à semences jaunes, ledit plant de *Brassica* à semences jaunes produisant des semences ayant une couleur jaune constante et stable.

2. Procédé pour la production d'un plant, d'une cellule végétale ou d'un tissu végétal de *Brassica* par croisement sélectif, ledit procédé comprenant :
a) le croisement d'une plante *B. carinata* à semences jaunes et d'une plante *B. campestris* à semences jaunes pour produire un haploïde trigénomique, le croisement de l'haploïde trigénomique avec *B. napus* et l'autofécondation de la descendance pour produire un plant de *Brassica* comme descendance ;
**caractérisé en ce qu'**il comprend en outre :
b) le croisement d'un premier plant de *Brassica* ayant un génome CC et d'un second plant de *Brassica* ayant un génome AA d'un *Brassica campestris* à semences jaunes, l'application d'une technique de sauvetage d'embryon avec ensuite une culture *in vitro* pour produire un plant de *Brassica* comme descendance ; et
c) le croisement du plant de *Brassica* obtenu comme descendance de l'étape (a) avec le plant de *Brassica* obtenu comme descendance de l'étape (b) et l'autofécondation des descendants pour produire un plant de *Brassica* à semences jaunes, ledit plant de *Brassica* à semences jaunes produisant des semences ayant une couleur jaune constante et stable.
